(19) Europäisches Patentamt
European Patent Office
Office européen des brevets

(11) Veröffentlichungsnummer: 0 231 900
A2

(12) # EUROPÄISCHE PATENTANMELDUNG

(21) Anmeldenummer: 87101293.6

(51) Int. Cl.⁴: C07C 127/22 , A01N 47/34

(22) Anmeldetag: 30.01.87

(30) Priorität: 01.02.86 DE 3603089

(43) Veröffentlichungstag der Anmeldung:
12.08.87 Patentblatt 87/33

(84) Benannte Vertragsstaaten:
AT BE CH DE FR GB IT LI NL

(71) Anmelder: BASF Aktiengesellschaft
Carl-Bosch-Strasse 38
D-6700 Ludwigshafen(DE)

(72) Erfinder: Freund, Wolfgang, Dr.
Johann-Gottlieb-Fichte-Strasse 71
D-6730 Neustadt(DE)
Erfinder: Parg, Adolf, Dr.
Paray-le-Monial-Strasse 8
D-6702 Bad Duerkheim(DE)
Erfinder: Adolphi, Heinrich, Dr.
Kalmitweg 11
D-6703 Limburgerhof(DE)
Erfinder: Hofmeister, Peter, Dr.
Bernard-Humblot-Strasse 12
D-6730 Neustadt(DE)

(54) **N-Benzoyl-N'-phenoxyphenylharnstoffe, Verfahren zu ihrer Herstellung und ihre Verwendung zur Bekämpfung von Schädlingen.**

(57) N-Benzoyl-N'-phenoxyphenylharnstoffe der Formel I

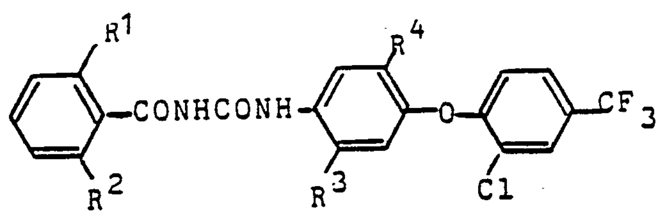

in der
$R^1$ Fluor oder Chlor
$R^2$ Fluor, Chlor oder Wasserstoff
$R^3$ Halogen
$R^4$ Wasserstoff oder Halogen bedeutet, Verfahren zu ihrer Herstellung und Verwendung zur Bekämpfung von Schadinsekten und Spinnentieren.

EP 0 231 900 A2

# N-Benzoyl-N'-phenoxyphenylharnstoffe, Verfahren zu ihrer Herstellung und ihre Verwendung zur Bekämpfung von Schädlingen

Die Erfindung betrifft N-Benzoyl-N'-phenoxyphenylharnstoffe, Verfahren zu ihrer Herstellung und Schädlingsbekämpfungsmittel, die diese Verbindungen als Wirkstoffe enthalten.

Es ist bekannt, daß N-Benzoyl-N'-phenoxyphenylharnstoffe als Insektizide verwendet werden können (J. Agr. Food Chem. 21, 348 (1973), DE-OS 2 537 413).

Aus der deutschen Offenlegungsschrift 25 37 413 sind N-Benzoyl-N'-phenoxyphenylharnstoffe der Formel A

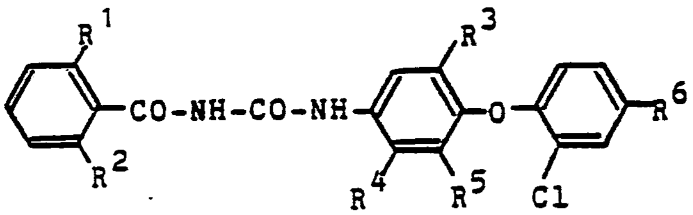

A,

bekannt, in der
$R^1$ für Wasserstoff, Chlor oder Fluor,
$R^2$ für Chlor oder Fluor,
$R^3$, $R^4$, $R^5$ für Wasserstoff und
$R^6$ für Trifluormethyl steht.

Die deutsche Offenlegungsschrift offenbart jedoch keine Verbindung der Formel I, in der $R^3$ für Halogen und gleichzeitig $R^4$ für Wasserstoff oder Halogen steht.

Außerdem sind aus den deutschen Offenlegungsschriften 25 37 413 und 25 28 917 Verbindungen der Formel A bekannt, in der
$R^1$ für Chlor, Fluor oder Brom,
$R^2$ für Wasserstoff oder Fluor,
$R^3$ für Wasserstoff,
$R^4$ für Methyl oder Chlor,
$R^5$ für Wasserstoff und
$R^6$ für Nitro
steht.

Jedoch beschreiben diese Offenlegungsschriften keine Verbindungen der Formel I, bei denen Trifluormethyl im Phenoxyrest in 4-Stellung steht.

Es wurden nun neue N-Benzoyl-N'-phenoxyphenylharnstoffe der Formel I gefunden

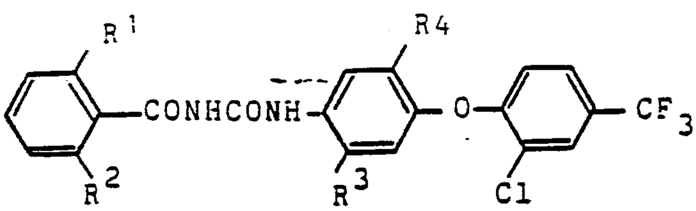

I,

in der
$R^1$ Fluor oder Chlor,
$R^2$ Fluor, Chlor oder Wasserstoff,
$R^3$ Halogen,
$R^4$ Wasserstoff oder Halogen bedeutet. Diese besitzen eine auffällig starke insektizide Wirkung.

Bevorzugte N-Benzoyl-N'-phenoxyphenylharnstoffe sind Verbindungen der Formel I, bei denen

$R^1$ Fluor oder Chlor,
$R^2$ Fluor, Chlor oder Wasserstoff,
$R^3$ Chlor oder Brom,
$R^4$ Wasserstoff, Chlor oder Brom bedeutet.

Überraschenderweise bekämpfen die erfindungsgemäßen N-Benzoyl-N'-phenoxyphenylharnstoffe Schädlinge wesentlich wirksamer als die aus dem Stand der Technik vorbeschriebenen Verbindungen analoger Konstitution und Wirkrichtung. Die erfindungsgemäßen Verbindungen stellen daher eine Bereicherung der Technik dar.

Man erhält die erfindungsgemäßen N-Benzoyl-N'-phenoxyphenylharnstoffe z.B. durch Umsetzung von einer entsprechenden Verbindung der Formel II

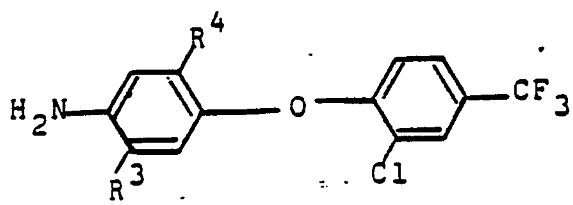 II,

mit einem entsprechenden Benzoylisocyanat der Formel III

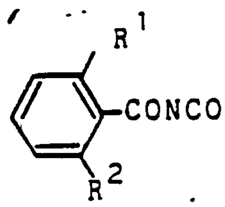 III,

oder eines entsprechenden Benzoesäureamids mit einem entsprechenden Phenoxyphenylisocyanat in jeweils an sich bekannter Weise.

Die erfindungsgemäßen Verbindungen sind in der Regel einheitliche, kristalline Stoffe mit definiertem Schmelzpunkt.

Wenn eine besondere Reinheit gefordert wird, können die üblichen Methoden zur Reinigung, z.B. Umkristallisation oder Chromatographie angewandt werden. Zu ihrer Charakterisierung dienen weiterhin Elementaranalyse, IR-und NMR-Spektrum.

Die Benzoylisocyanate III sind bekannt. Man kann sie nach den Vorschriften in J. Org. Chem. 28 , 1805 -1811 (1963), J. Agr. Food Chem. 21, 348 (1973) oder der europäischen Patentschrift 143 302 erhalten.

Die Phenoxyaniline II können nach allgemein üblichen Verfahren hergestellt werden, beispielsweise aus einem Alkaliphenolat und 1,2-Dichlor-4-trifluormethylbenzol, Nitrierung der Reaktionsprodukte und Reduktion:

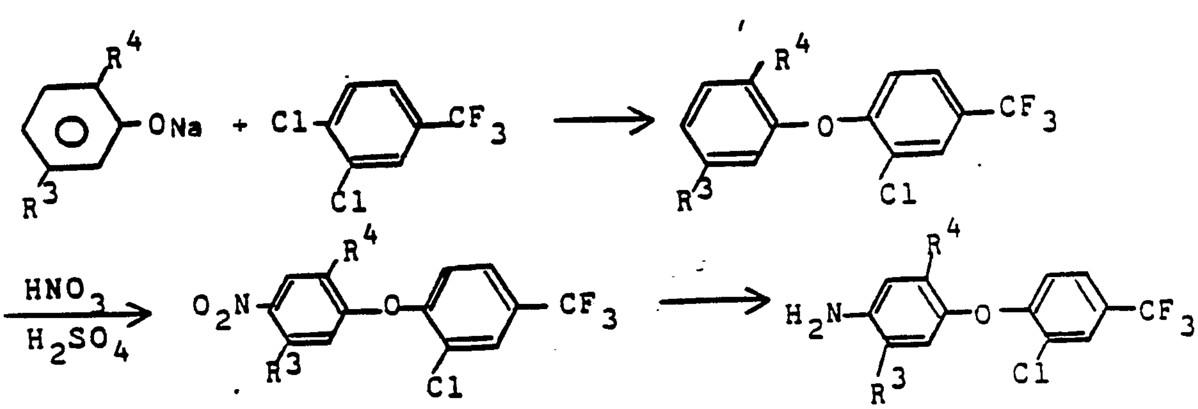


(II)

Falls man sich für die Umsetzung von Benzoesäureamiden mit Phenoxyphenylisocyanaten entscheidet, können diese in bekannter Weise aus den Anilinen der Formel II z.B. durch Umsetzung mit Phosgen erhalten werden (vgl. z.B. EP 98 158).

Herstellungsbeispiele:

1. N-(2-Fluorbenzoyl)-N'-[4-(2-chlor-4-trifluormethylphenoxy)-2,5-dichlor-phenyl]harnstoff (Wirkstoff Nr. 1)

7.0 g 2,5-Dichlor-4-(2-chlor-4-trifluormethylphenoxy)anilin werden bei Raumtemperatur in 50 ml wasserfreiem Toluol gelöst. Man gibt 1 Tropfen Triethylamin hinzu und tropft 3,3 g 2-Fluorbenzoylisocyanat in 10 ml wasserfreiem Toluol zu. Man rührt noch 3 Stunden bei Raumtemperatur nach, saugt ab und wäscht mit Pentan. Ausbeute 9,5 g mit einem Schmelzpunkt von 191 -194°C

2. N-(2,6-Difluorbenzoyl)-N'-[4-(2-chlor-4-trifluormethylphenoxy)-2,5-dichlorphenyl]harnstoff (Wirkstoff Nr. 2)

89 g 2,5-Dichlor-4-(2-chlor-4-trifluormethylphenoxy)anilin werden bei Raumtemperatur in 200 ml wasserfreiem Acetonitril gelöst und 27,5 g 2,6-Difluorbenzoylisocyanat in 60 ml wasserfreiem Acetonitril zugesetzt. Man rührt 14 Stunden bei Raumtemperatur nach und saugt ab und trocknet. Ausbeute 53 g mit einem Schmelzpunkt von 175 -180°C.

3. N-(2,6-Dichlorbenzoyl)-N'-[4-(2-chlor-4-trifluormethylphenoxy) -2-chlorphenyl]harnstoff (Wirkstoff Nr. 3)

10,0 g 2-Chlor-4-(3-chlor-4-trifluormethylphenoxy)anilin werden bei Raumtemperatur in 40 ml wasserfreiem Toluol gelöst. Man gibt 1 Tropfen Triethylamin hinzu und setzt eine Lösung von 6,9 g 2,6-Dichlorbenzoylisocyanat in 20 ml wasserfreiem Toluol zu. Man rührt 3 Stunden bei Raumtemperatur nach, zieht das Lösungsmittel im Rotationsverdampfer ab und chromatographiert mit Toluol/Aceton an Kieselgel. Nach dem Einengen erhält man 13 g der Titelverbindung mit einem Schmelzpunkt von 142 -150°C.

4. Herstellungsbeispiel für die Phenoxyaniline der Formel II:

Schritt a:

Zu 201 g Kalium-2,5-dichlorphenolat in 800 ml N-Methylpyrrolidon tropft man 430 g 1,2-Dichlor-4-trifluormethylbenzol und erhitzt 15 Stunden auf 160°C. Man gießt auf 5 l Wasser, trennt die organische Phase ab, trocknet mit Magnesiumsulfat und destilliert unter vermindertem Druck (Ölpumpe). Man erhält 447 g 2,5-Dichlor-2'-chlor-4'-trifluormethyldiphenylether, Siedepunkt 125 -135°C, $n_D^{25}$ 1,5499.

Schritt b:

Zu 405 g des Diphenylethers in 600 ml Essigsäureanhydrid tropft man bei 0 -10°C eine Mischung aus 123 ml 65 % Salpetersäure und 92 ml konz. Schwefelsäure. Man rührt 2 Stunden bei Raumtemperatur nach, gießt in 5 l Eiswasser und saugt ab. Nach Umkristallisation aus Isopropanol erhält man 463 g 2,5-Dichlor-4-nitro-2'-chlor-4'-trifluormethyl-diphenylether mit einem Schmelzpunkt von 88 -95°C.

Schritt c:

Zu einer Suspension von 19,6 g Eisenpulver in einer siedenden Mischung von 250 g Methanol und 50 g Eisessig tropft man eine Lösung aus 38,7 g der Nitroverbindung in 190 ml Eisessig. Man hält weitere 2 Stunden am Rückfluß und gießt in 3 l Wasser. Die abgeschiedene ölige Flüssigkeit wird in Essigsäureethylester aufgenommen, mit Magnesiumsulfat getrocknet und eingeengt. Man erhält 35 g 2,5-Dichlor-4-(2-chlor-4-trifluormethylphenoxy)anilin mit einem Schmelzpunkt von 79 -87°C.

Die in der nachstehenden Tabelle mit ihren Schmelzpunkten angegebenen Verbindungen wurden auf entsprechende Weise hergestellt; die übrigen Verbindungen der Tabelle können unter entsprechender Abwandlung der Herstellvorschriften erhalten werden.

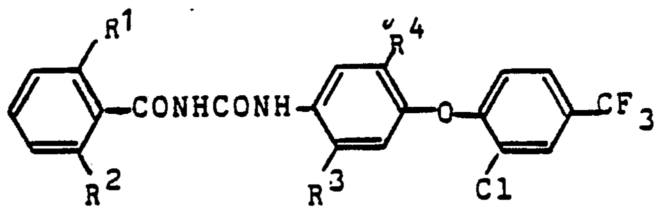

| Wirkstoff Nr. | $R^1$ | $R^2$ | $R^3$ | $R^4$ | Fp/°C |
|---|---|---|---|---|---|
| 4 | F | H | F | H | |
| 5 | F | H | F | F | |
| 6 | F | H | F | Cl | |
| 7 | F | H | F | Br | |
| 8 | F | H | Cl | H | 190 - 198 |
| 9 | F | H | Cl | F | |
| 10 | F | H | Cl | Br | |
| 11 | F | H | Br | H | 185 - 192 |
| 12 | F | H | Br | F | |
| 13 | F | H | Br | Cl | |
| 14 | F | H | Br | Br | |
| 15 | Cl | H | F | H | |
| 16 | Cl | H | F | F | |
| 17 | Cl | H | F | Cl | |
| 18 | Cl | H | F | Br | |
| 19 | Cl | H | Cl | H | 172 - 176 |
| 20 | Cl | H | Cl | F | |

Fortsetzung der Tabelle:

| Wirkstoff Nr. | $R^1$ | $R^2$ | $R^3$ | $R^4$ | Fp/°C |
|---|---|---|---|---|---|
| 21 | Cl | H | Cl | Cl | 193 - 196 |
| 22 | Cl | H | Cl | Br | |
| 23 | Cl | H | Br | H | 169 - 171 |
| 24 | Cl | H | Br | F | |
| 25 | Cl | H | Br | Cl | |
| 26 | Cl | H | Br | Br | |
| 27 | Cl | Cl | F | H | |
| 28 | Cl | Cl | F | F | |
| 29 | Cl | Cl | F | Cl | |
| 30 | Cl | Cl | F | Br | |
| 31 | Cl | Cl | Cl | F | |
| 32 | Cl | Cl | Cl | Cl | 208 - 213 |
| 33 | Cl | Cl | Cl | Br | |
| 34 | Cl | Cl | Br | H | 109 - 112 |
| 35 | Cl | Cl | Br | F | |
| 36 | Cl | Cl | Br | Cl | |
| 37 | Cl | Cl | Br | Br | |
| 38 | F | F | F | H | |
| 39 | F | F | F | F | |
| 40 | F | F | F | Cl | |
| 41 | F | F | F | Br | |
| 42 | F | F | Cl | H | 134 - 139 |
| 43 | F | F | Cl | F | |
| 44 | F | F | Cl | Br | |
| 45 | F | F | Br | H | 140 - 148 |
| 46 | F | F | Br | F | |
| 47 | F | F | Br | Cl | |
| 48 | F | F | Br | Br | |

Für die nachstehenden Anwendungsbeispiele wurden die folgenden Vergleichsmittel gewählt:

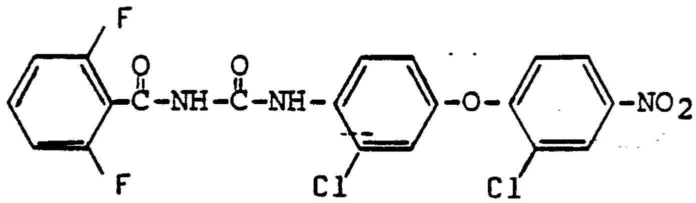

(DE-OS 2528917, Beisp. 3)
(DE-OS 2537413, " 15)
Vergleichsmittel I

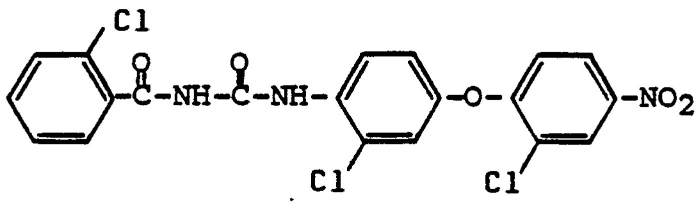

(DE-OS 2528917, Beisp. 1)
(DE-OS 1537413, " 13)
**Vergleichsmittel II**

Die Ergebnisse sind in den folgenden Tabellen aufgelistet.

Entwicklungshemmung bei Prodenia litura

(Prüfung auf behandelten Mais-Blättern)

Junge Maisblätter taucht man für 5 Sekunden in die wäßrige Wirkstoffaufbereitung. Nach kurzem Antrocknen der Beläge gibt man die Blätter auf einen 9 cm Rundfilter in Petrischalen (∅ 10 cm) und besetzt sie mit je 5 L 3-Raupen (1,5 -1,8 cm).
Die Schalen lagern bei 23°C.
Nach 3 Tagen setzt man die Raupen auf unbehandeltes Kunstfutter. Beurteilt wird Verpuppung und Schlupf der Falter.
Ergebnis:

| | | | | |
|---|---|---|---|---|
| Beispiel Nr. | 1 | 0,1 | ppm | 100 % Mort. |
| " | 2 | 0,04 | ppm | 100 % Mort. |
| " | 3 | 0,4 | ppm | 100 % Mort. |
| " | 8 | 0,4 | ppm | 100 % Mort. |
| " | 19 | 0,04 | ppm | 100 % Mort. |
| " | 21 | 0,04 | ppm | 100 % Mort. |

| | | | | |
|---|---|---|---|---|
| Beispiel Nr. | 23 | 0,04 | ppm | 100 % Mort. |
| " | 32 | 0,2 | ppm | 100 % Mort. |
| " | 42 | 0,04 | ppm | 100 % Mort. |
| " | 45 | 0,04 | ppm | 100 % Mort. |
| Vergleichsmittel | I | 0,1 | ppm | 100 % Mort. |
| " | II | 1,0 | ppm | ≤ 20 % Mort. |

(Konzentrationen unter 0,04 ppm wurden nicht untersucht.)

Entwicklungshemmung bei Heliothis virescens

(Prüfung auf behandeltem Nährboden)

100 g des Standard-Nährbodens für Heliothis werden in 150 ml-Becher gefüllt und warm mit der wäßrigen Wirkstoffaufbereitung sorgfältig gemischt.

Nach Erkalten belegt man jedes Gefäß mit 10 L 3-Raupen (1,5 -1,8 cm) und lagert sie bei 23°C. Beurteilt wird Verpuppung und Schlupf der Falter.

Ergebnis:

| | | | | |
|---|---|---|---|---|
| Beispiel Nr. | 1 | 10 | ppm | 100 % Mort. |
| " | 2 | 0,1 | ppm | 95 % " |
| " | 3 | 4 | ppm | 100 % " |
| " | 19 | 1 | ppm | 87 % " |
| " | 21 | 0,1 | ppm | 83 % " |
| " | 32 | 4 | ppm | 87 % " |
| " | 42 | 0,1 | ppm | 83 % " |
| " | 45 | 0,1 | ppm | 83 % " |
| Vergleichsmittel | I | 0,1 | ppm | 79 % " |
| Vergleichsmittel | II | 40 | ppm | 100 % " |

Entwicklungshemmung bei Agrotis ypsilon

(Prüfung auf behandelten Maisblättern)

Junge Maisblätter taucht man für 5 Sekunden in die wäßrige Wirkstoffaufbereitung. Nach kurzem Antrocknen der Beläge gibt man die Blätter auf einen 9 cm Rundfilter in Petrischalen (Ø 10 cm) und besetzt sie mit je 5 L 3-Raupen (1,5 -1,8 cm).

Die Schalen lagern bei 23°C.

Nach 3 Tagen setzt man die Raupen auf unbehandeltes Kunstfutter. Beurteilt wird Verpuppung und Schlupf der Falter.

Ergebnis:

| | | | |
|---|---|---|---|
| Beispiel Nr. | 2 | 0,1 ppm | 90 % Mort. |
| " | 3 | 4 ppm | 95 % " |
| " | 19 | 1 ppm | 100 % " |
| " | 21 | 0,4 ppm | 100 % " |
| " | 23 | 4 ppm | 93 % " |
| " | 32 | 4 ppm | 90 % " |
| " | 45 | 0,4 ppm | 97 % " |
| Vergleichsmittel | I | 0,1 ppm | 79 % " |
| " | II | 40 ppm | 100 % " |

Zuchtversuch mit Stubenfliege (Musca domestica)

Als Versuchsgefäße dienen 50 ml Penicillingläser. Diese werden mit je 4,75 ml Nährbrei beschickt und mit 0,25 ml wäßriger Wirkstoffaufbereitung versetzt.

In einem Becherglas schwämmt man ca. 10 000 einen Tag alte Fliegeneier in ca. 100 ml Leitungswasser auf. Aus dieser Suspension überträgt man mittels einer Pipette je einen Tropfen in ein Glas.

Der fertige Versuchsansatz wird mit einem Filterpapier abgedeckt und bei 23 -24°C gelagert.

Ergebnis:

| | | | |
|---|---|---|---|
| Beispiel Nr. | 2 | < 1 ppm | 100 % Mort. |
| " | 42 | 4 ppm | 100 % " |
| Vergleichsmittel | I | 20 ppm | ca. 80 % " |
| " | II | 50 ppm | < 50 % " |

Zuchtversuch, Mittelmeerfliege-Fruchfliege (Ceratitis capitata)

Die Versuche werden in 100 ml Plastik-Bechern auf 40 g eines Nährbodens aus Karottenpulver und Wasser 1 : 3 mit Hefezusatz durchgeführt.

Der Wirkstoff wird als wäßrige Aufbereitung in den fertigen Brei eingerührt und dieser mit 100 -200 frischen Eiern beimpft. Es ist darauf zu achten, daß die wäßrige Eier-Aufschlämmung sehr schnell verwendet wird, da sonst die Larven bereits schlüpfen und absterben.

Die Becher werden bei 24 -26°C geschlossen aufbewahrt; nach ca. einer Woche kann die Entwicklung beurteilt werden.

Ergebnis:

| | | | |
|---|---|---|---|
| Beispiel Nr. | 2 | 4 ppm | ca. 80 % Mort. |
| " | 42 | 10 ppm | 100 % " |
| " | 45 | 20 ppm | ca. 80 % " |
| Vergleichsmittel | I | 10 ppm | ca. 80 % " |
| Vergleichsmittel | II | 20 ppm | < 50 % " |

Zuchtversuch mit Baumwollwanzen (Dysdercus intermedius)

50 g Baumwollsaat werden für 24 Stunden in der wäßrigen Aufbereitung von 10 Gew.-% Wirkstoff und 90 Gew.-% eines Emulgatorgemisches aus 10 % ethoxyliertem Rizinusöl, 20 % ethoxyliertem Isooctylphenol und 70 % Cyclohexanon eingequollen. Die überstehende Flüssigkeit wird abgeschüttet.

Es wird beobachtet, ob die Tiere
a) überleben und
b) sich zu Adulten häuten. Ergebnis:

| | | | |
|---|---|---|---|
| Beispiel Nr. | 2 | 2 ppm | 100 % Mort. |
| " | 42 | 10 ppm | 100 % " |
| " | 45 | 2 ppm | 100 % " |
| Vergleichsmittel I | | 10 ppm | < 50 % " |
| Vergleichsmittel II | | 10 ppm | < 50 % " |

Versuche mit gezählten Weibchen der Spinnmilbe (Tetranychus telarius)

Getopfte Buschbohnen werden in der Spritzkabine auf dem Drehteller mit der wäßrigen Wirkstoffaufbereitung tropfnaß gespritzt. Nach dem Abtrocknen stanzt man mit einem Korkbohrer Blattstücke von 25 mm Durch messer aus den Spreiten. Sie werden auf Zellstoffstücke gelegt, welche an den Seiten im Wasser hängen.

Die Blattstanzstücke werden mit je 10 adulten Weibchen belegt. Nach 10 Tagen erfolgt die Auswertung, wobei die lebenden Larven ausgezählt werden.
Ergebnis:

| | | | |
|---|---|---|---|
| Beispiel Nr. | 2 | 0,02 ppm | ca. 90 % Mort. |
| Vergleichsmittel I | | 0,1 ppm | ca. 75 % " |
| Vergleichsmittel II | | 0,1 ppm | ca. 30 % " |

**Ansprüche**

1. N-Benzoyl-N'-phenoxyphenylharnstoffe der Formel I

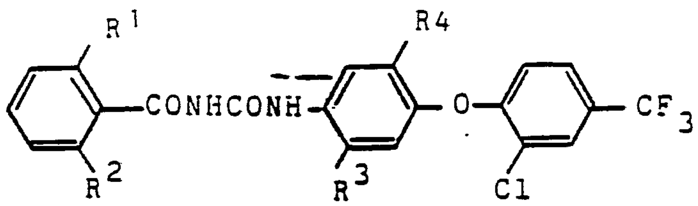

I,

in der
R' Fluor oder Chlor

$R^2$ Fluor, Chlor oder Wasserstoff
$R^3$ Halogen
$R^4$ Wasserstoff oder Halogen bedeutet.

2. Verfahren zur Herstellung von N-Benzoyl-N'-phenoxyphenylharnstoffen der Formel I gemäß Anspruch 1, dadurch gekennzeichnet, daß man ein entsprechendes Phenoxyanilin der Formel II

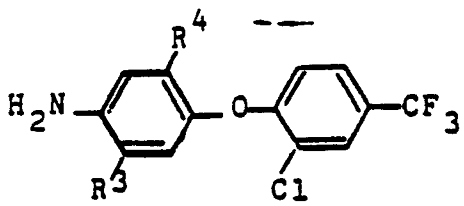 II,

mit einem entsprechenden Benzoylisocyanat der Formel III

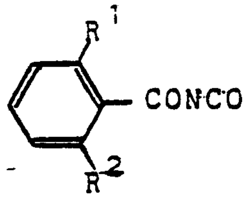 III,

umsetzt.